Europäisches Patentamt

European Patent Office

Office européen des brevets

(19)

(11) Veröffentlichungsnummer: **0 210 680**
**B1**

(12) **EUROPÄISCHE PATENTSCHRIFT**

(45) Veröffentlichungstag der Patentschrift:
25.01.89

(21) Anmeldenummer: 86201123.6

(22) Anmeldetag: 26.06.86

(51) Int. Cl.⁴: **B01J 8/06**
// C07C63/16, C07C15/46,
C07C51/265, C07C51/31

(54) Röhrenreaktor.

(30) Priorität: 27.07.85 DE 3526967

(43) Veröffentlichungstag der Anmeldung:
04.02.87 Patentblatt 87/6

(45) Bekanntmachung des Hinweises auf die Patenterteilung:
25.01.89 Patentblatt 89/4

(84) Benannte Vertragsstaaten:
DE FR GB IT NL

(56) Entgegenhaltungen:
FR-A- 1 531 940
FR-A- 2 118 099

(73) Patentinhaber: METALLGESELLSCHAFT AG,
Reuterweg 14 Postfach 3724, D-6000 Frankfurt/M.1(DE)

(72) Erfinder: Franz, Volker, Kronberger Strasse 37,
D-6000 Frankfurt am Main(DE)
Erfinder: Geissen, Rolf, Ginnheimer Strasse 25,
D-6000 Frankfurt am Main(DE)

(74) Vertreter: Rieger, Harald, Dr., Reuterweg 14,
D-6000 Frankfurt am Main(DE)

## Beschreibung

Die Erfindung betrifft einen Röhrenreaktor für exotherme oder endotherme Umsetzungen in zahlreichen Röhren, die von einer Flüssigkeit umgeben sind, welche man über eine außerhalb des Reaktors angeordnete Umwälzpumpe und eine Erhitzung oder Kühlung mindestens teilweise im Kreislauf zurück zum Reaktor führt.

Röhrenreaktoren dieser Art sind bekannt und z.B. in der deutschen Offenlegungsschrift 2 846 693 und dem dazu korrespondierenden US-Patent 4 263 141 beschrieben. Die bekannten Röhrenreaktoren enthalten in den Röhren einen körnigen Katalysator in Form einer Schüttung, durch die ein umzusetzendes Gasgemisch geleitet wird. Die Röhren sind im allgemeinen auf konzentrischen Kreisen angeordnet, wobei das Verhältnis der Durchmesser von äußerstem und innerstem Kreis je nach Bauart des Reaktors etwa zwischen 10:1 und 20:1 schwankt. Die Flüssigkeit, die Wärme zu- oder abführt, muß auf ihrem Strömungsweg entlang der Röhren viele Rohrreihen passieren, wodurch ein hoher Druckverlust entsteht. Auch sind die Strömungsgeschwindigkeiten für die Flüssigkeiten beim Passieren der inneren Rohrreihen aufgrund der großen Durchmesserunterschiede sehr viel größer als bei den äußeren Rohrreihen, woraus sich sehr unterschiedliche Wärmeübergangswerte ergeben. Um entlang der äußeren Röhren noch eine ausreichende Wärmeübertragung zu gewährleisten, muß die umgewälzte Flüssigkeitsmenge groß gewählt werden. Dadurch steigt aber die Strömungsgeschwindigkeit der Flüssigkeit im Bereich der inneren Rohrreihen in unerwünschter Weise an. Zum Nachteil der unterschiedlichen Wärmeübertragung kommt also auch noch ein hoher Leistungsbedarf für die Umwälzung der Flüssigkeit hinzu.

Bei den bekannten Röhrenreaktoren mit außenliegender Umwälzpumpe wird die Flüssigkeit, bevor sie in den Reaktor eintritt, zunächst in eine ringförmige Sammelleitung eingeleitet, die an der Außenwand des Reaktors angeordnet ist. Von dieser Sammelleitung führen zahlreiche Öffnungen in den von den Röhren durchzogenen Bereich des Reaktors. Der Rückfluß zur Umwälzpumpe geschieht in gleicher Weise durch eine weitere Sammelleitung an der Außenwand des Reaktors. Die Querschnitte der außenliegenden Sammelleitungen müssen für etwa die halbe Menge an umzuwälzender Flüssigkeit ausgelegt werden, auch sind die Verbindungsleitungen zwischen den Sammelkanälen und der Umwälzpumpe häufig von erheblicher Länge. Will man mit kurzen Leitungen auskommen, so kann man dies in bekannter Weise dadurch, daß man anstelle einer Pumpe zwei Pumpen verwendet, was jedoch auch einen störenden Aufwand bedeutet.

Der Erfindung liegt die Aufgabe zugrunde, einen Röhrenreaktor so auszubilden, daß die Flüssigkeit im Bereich der Röhren wenig Strömungswiderstand vorfindet, daß eine möglichst gleichmäßige Zufuhr bzw. Abfuhr von Wärme erreicht wird und kurze Verbindungsleitungen zwischen Reaktor und Umwälzpumpe möglich werden. Erfindungsgemäß geschieht dies beim eingangs genannten Röhrenreaktor dadurch, daß sich die Röhren und die umgebende Flüssigkeit zwischen einer etwa zylindrischen Innenwand und einer die Innenwand etwa koaxial umgebenden Außenwand befinden und daß die Umwälzpumpe in dem von der Innenwand umgebenen, flüssigkeitsfreien Raum angeordnet ist. Dadurch, daß sich die Pumpe in einem flüssigkeitsfreien Raum befindet, ist sie von außen, z.B. für Wartungsarbeiten, leicht zugänglich. Bei den im allgemeinen entlang Kreisen angeordneten Röhren des erfindungsgemäßen Reaktors ist das Durchmesserverhältnis von äußerstem zu innerstem Kreis viel kleiner als bei bekannten Röhrenreaktoren.

Es ist vorteilhaft, daß an der Innenwand des erfindungsgemäßen Röhrenreaktors mindestens je eine Sammelleitung zum Einspeisen von Flüssigkeit in den Reaktor und zum Abziehen von Flüssigkeit aus dem Reaktor angeordnet ist. Zweckmäßigerweise werden zwischen dem Reaktor und der Umwälzpumpe mindestens je zwei Verbindungsleitungen zum Einspeisen und Abziehen von Flüssigkeit vorgesehen. Die Sammelleitungen sind hierdurch über mehrere Leitungen, z.B. sternförmig, an die Umwälzpumpe angeschlossen, so daß der Querschnitt der Sammelleitungen gegenüber einer Ausführungsform mit nur einer Verbindungsleitung verkleinert werden kann.

Der erfindungsgemäße Röhrenreaktor weist vorzugsweise einen Durchmesser der Innenwand von mindestens 1,2 m und einen Durchmesser der Außenwand von mindestens 4 m auf. Bei solchen großen Reaktoren kommen seine Vorteile besonders gut zur Geltung. Zumeist liegt das Verhältnis der Durchmesser der Außenwand und der Innenwand im Bereich von 4:1 bis 1,1:1 und vorzugsweise im Bereich von 3:1 bis 1,5:1. Selbstverständlich kann man nicht nur die Pumpe, sondern auch die Erhitzung oder Kühlung im von der Innenwand umgebenen Raum anordnen, wenn eine kompakte Bauweise gewünscht wird.

Ausgestaltungsmöglichkeiten des Reaktors werden mit Hilfe der Zeichnung erläutert. Es zeigt:

Figur 1 einen Längsschnitt durch den Reaktor in teilweise schematisierter Darstellung und
Figur 2 einen Querschnitt durch den Reaktor der Figur 1 entlang der Linie II–II.

Der Röhrenreaktor besitzt eine zylindrische Innenwand 1 und eine dazu etwa koaxiale Außenwand 2. Die Außenwand 2 ist im oberen und unteren Bereich nach innen umgebogen und dort mit der Innenwand 1 verbunden. Dabei entsteht ein oberer Verteilerraum 3, dem das umzusetzende Gasgemisch durch die Leitung 4 zugeführt wird, sowie ein unterer Verteilerraum 5, aus dem man das Produkt durch die Leitung 6 abzieht. Zwischen einem oberen Rohrboden 7 und einem unteren Rohrboden 8 sind zahlreiche senkrechte Röhren 10 angeordnet, die üblicherweise mit körnigem Katalysatormaterial gefüllt sind. Das umzusetzende Gemisch gelangt in bekannter Weise vom oberen Verteilerraum 3 in die Röhren 10, durchströmt diese, wobei eine endotherme oder exotherme Umsetzung erfolgt und das Produkt über den unteren Verteilerraum 5 und die Leitung 6 abzieht.

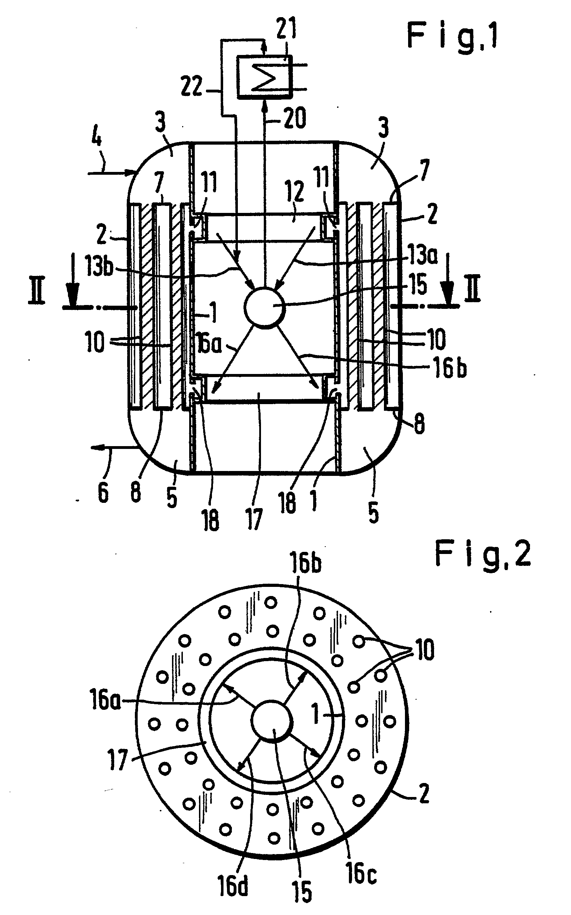

Um Wärme aus dem Innenbereich der Röhren 10 abzuführen bzw. Wärme diesem Bereich zuzuführen, sind die Röhren 10 von einer Flüssigkeit umgeben, die im Kreislauf geführt wird. Diese Flüssigkeit gelangt zunächst durch Öffnungen 11 in der Innenwand 1 zu einer oberen Sammelleitung 12 und von da über mehrere Verbindungsleitungen 13a und 13b zur Umwälzpumpe 15. Die Umwälzpumpe 15 befindet sich im flüssigkeitsfreien Bereich, der von der Innenwand 1 umgeben ist. Ein Teilstrom der durch die Leitungen 13a und 13b angesaugten Flüssigkeit wird durch die Verbindungsleitungen 16a, 16b, 16c und 16d, vergl. auch Figur 2, einer unteren Sammelleitung 17 zugeführt. Die untere Sammelleitung 17 ist wie die obere Sammelleitung 12 ringförmig ausgebildet und liegt an der Innenwand 1 an, wobei sie durch Öffnungen 18 mit dem von den Röhren 10 durchzogenen Bereich des Reaktors verbunden ist. Durch diese Öffnungen 18 tritt die von der Pumpe 15 herangeführte Flüssigkeit in den Reaktor ein, umströmt die Röhren 10 und wird über die obere Sammelleitung 12 wieder von der Umwälzpumpe 15 angesaugt.

Ein Teilstrom der Flüssigkeit wird ständig von der Pumpe 15 durch die Leitung 20 dem Wärmeaustauscher 21 zugeführt und von dort durch die Leitung 22 in den Ansaugbereich der Pumpe 15 zurückgeleitet. Im Wärmeaustauscher 21 erfolgt die Erhitzung oder Kühlung der Flüssigkeit, je nachdem, ob die Flüssigkeit Wärme an die Röhren 10 abgeben oder von diesen abführen soll.

Selbstverständlich kann der Wärmeaustauscher 21 auch innerhalb des von der Innenwand 1 umgebenen flüssigkeitsfreien Bereichs angeordnet sein, was jedoch in Figur 1 der besseren Übersichtigkeit wegen nicht berücksichtigt wurde.

Bei der Flüssigkeit kann es sich z.B. um Wasser, eine Salzschmelze oder aber um eine an sich bekannte organische Flüssigkeit handeln. Jedenfalls ist der Röhrenreaktor nicht auf die Verwendung einer bestimmten Flüssigkeit eingeschränkt. Die Zahl der Verbindungsleitungen zwischen der oberen Sammelleitung 12 und der Pumpe 15 sowie zwischen der Pumpe 15 und der unteren Sammelleitung 17 ist nicht beschränkt und liegt meist zwischen 2 und 6.

Durch den erfindungsgemäßen Röhrenreaktor wird eine wesentliche Energieeinsparung für den Antrieb der Umwälzpumpe 15 erreicht, ferner wird die Wärmeübertragung zwischen der Flüssigkeit und den Röhren vergleichmäßigt. Weitere Vorteile bestehen darin, daß die Umwälzmenge der Flüssigkeit gegenüber bekannten Röhrenreaktoren bei gleicher Pumpenleistung erhöht werden kann, wodurch zusätzlich eine gleichmäßigere Temperaturverteilung in horizontaler und vertikaler Richtung im Reaktor erreicht wird, was für viele Prozesse von ausschlaggebender Bedeutung ist. Auch bei sehr großen Reaktoren wird nicht mehr als eine Umwälzpumpe benötigt. Nur als Beispiele für die Anwendungsmöglichkeiten des Reaktors seien hier die Herstellung von Phthalsäureanhydrid aus Orthoxylol oder Naphthalin oder aber die Dehydrierung von Äthylbenzol zu Styrol genannt.

**Patentansprüche**

1. Röhrenreaktor für exotherme oder endotherme Umsetzungen in zahlreichen Röhren, die von einer Flüssigkeit umgeben sind, welche man über eine außerhalb des Reaktors angeordnete Umwälzpumpe und eine Erhitzung oder Kühlung mindestens teilweise im Kreislauf zurück zum Reaktor führt, dadurch gekennzeichnet, daß sich die Röhren und die umgebende Flüssigkeit zwischen einer etwa zylindrischen Innenwand und einer die Innenwand etwa koaxial umgebenden Außenwand befinden und daß die Umwälzpumpe in dem von der Innenwand umgebenen, flüssigkeitsfreien Raum angeordnet ist.

2. Röhrenreaktor nach Anspruch 1, dadurch gekennzeichnet, daß an der Innenwand mindestens je eine Sammelleitung zum Einspeisen von Flüssigkeit in den Reaktor und zum Abziehen von Flüssigkeit aus dem Reaktor angeordnet ist.

3. Röhrenreaktor nach Anspruch 1 oder 2, dadurch gekennzeichnet, daß zwischen dem Reaktor und der Umwälzpumpe mindestens je zwei Verbindungsleitungen zum Einspeisen und Abziehen von Flüssigkeit bestehen.

4. Röhrenreaktor nach Anspruch 1 oder einem der folgenden, dadurch gekennzeichnet, daß der Durchmesser der Innenwand mindestens 1,2 m und der Durchmesser der Außenwand mindestens 4 m beträgt.

5. Röhrenreaktor nach Anspruch 1 oder einem der folgenden, dadurch gekennzeichnet, daß das Verhältnis der Durchmesser der Außenwand und der Innenwand im Bereich von 4:1 bis 1,1:1 und vorzugsweise im Bereich von 3:1 bis 1,5:1 liegt.

6. Röhrenreaktor nach Anspruch 1 oder einem der folgenden, dadurch gekennzeichnet, daß auch die Erhitzung oder Kühlung im von der Innenwand umgebenen Raum angeordnet ist.

**Claims**

1. Multi-tube reactor for exothermic or endothermic reactions in multiple tubes which are surrounded by a liquid which is circulated at least partly via a circulating pump disposed outside the reactor and a heating or cooling device back to the reactor, characterised in that the tubes and the surrounding liquid are located between an approximately cylindrical inner wall and an outer wall surrounding the inner wall approximately coaxially, and that the circulating pump is disposed in the liquid-free space surrounded by the inner wall.

2. Multi-tube reactor according to claim 1, characterised in that at least one manifold each for supplying liquid to the reactor and for removing liquid from the reactor are disposed on the inner wall.

3. Multi-tube reactor according to claim 1 or 2, characterised in that there are at least two connection lines each for supplying and for removing liquid between the reactor and the circulating pump.

4. Multi-tube reactor according to claim 1 or one of the following claims, characterised in that the diameter of the inner wall is at least 1.2 m and the diameter of the outer wall is at least 4 m.

5. Multi-tube reactor according to claim 1 or one of the following claims, characterised in that ratio of the diameters of the outer wall and the inner wall is in the range 4:1 to 1.1:1 and preferably in the range 3:1 to 1.5:1.

6. Multi-tube reactor according to claim 1 or one of the following claims, characterised in that the heating or cooling device is also disposed in the space surrounded by the inner wall.

**Revendications**

1. Réacteur tubulaire pour effectuer des réactions exothermiques ou endothermiques dans un très grand nombre de tubes qui sont entourés d'un liquide que l'on retourne, au moins partiellement, en circuit fermé, au réacteur, en passant par une pompe de circulation disposée à l'extérieur du réacteur et par un dispositif de chauffage ou de refroidissement, caractérisé en ce que les tubes et le liquide qui les entoure se trouvent entre une paroi intérieure sensiblement cylindrique et une paroi extérieure entourant sensiblement coaxialement la paroi intérieure, et en ce que la pompe de circulation est disposée dans l'espace exempt de liquide qui est entouré par la paroi intérieure.

2. Réacteur tubulaire suivant la revendication 1, caractérisé en ce que, sur la paroi intérieure, sont montés respectivement des conduits collecteurs d'alimentation du réacteur en liquide et de soutirage du liquide du réacteur.

3. Réacteur tubulaire suivant la revendication 1 ou 2, caractérisé en ce que, entre le réacteur et la pompe de circulation, sont prévus au moins respectivement deux conduits de liaison pour l'alimentation en liquide et pour son soutirage.

4. Réacteur tubulaire suivant la revendication 1 ou l'une des suivantes, caractérisé en ce que le diamètre de la paroi intérieure est d'au moins 1,2 m et le diamètre de la paroi extérieure est d'au moins 4 m.

5. Réacteur tubulaire suivant la revendication 1 ou l'une des suivantes, caractérisé en ce que le rapport du diamètre extérieur de la paroi extérieure à celui de la paroi intérieure est de l'ordre de 4:1 à 1,1:1 et, de préférence, de l'ordre de 3:1 à 1,5:1.

6. Réacteur tubulaire suivant la revendication 1 ou l'une des suivantes, caractérisé en ce que le dispositif de chauffage du dispositif de refroidissement est disposé aussi dans l'espace entouré par la paroi intérieure.

Fig.1

Fig.2